(19) Europäisches Patentamt
European Patent Office
Office européen des brevets

(11) **EP 1 658 042 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**25.03.2015 Patentblatt 2015/13**

(45) Hinweis auf die Patenterteilung:
**16.11.2011 Patentblatt 2011/46**

(21) Anmeldenummer: **04764198.0**

(22) Anmeldetag: **17.08.2004**

(51) Int Cl.:
*A61K 8/25* (2006.01)    *A61K 8/22* (2006.01)
*A61Q 5/08* (2006.01)

(86) Internationale Anmeldenummer:
**PCT/EP2004/009208**

(87) Internationale Veröffentlichungsnummer:
**WO 2005/020945 (10.03.2005 Gazette 2005/10)**

(54) **Milde Bleichmittel mit erhöhter Aufhelleistung**

Mild bleaching agent with increased lightening power

Agent de blanchiment doux à pouvoir éclaircissant accru

(84) Benannte Vertragsstaaten:
**AT BE BG CH CY CZ DE DK EE ES FI FR GB GR HU IE IT LI LU MC NL PL PT RO SE SI SK TR**

(30) Priorität: **26.08.2003 DE 10339163**

(43) Veröffentlichungstag der Anmeldung:
**24.05.2006 Patentblatt 2006/21**

(73) Patentinhaber: **Henkel AG & Co. KGaA**
**40589 Düsseldorf (DE)**

(72) Erfinder:
• **POPPE, Elisabeth**
**22763 Hamburg (DE)**
• **HÖFFKES, Horst**
**40595 Düsseldorf (DE)**

(56) Entgegenhaltungen:
**EP-A- 1 219 285     WO-A-01/28508**
**WO-A-01/47486     WO-A-01/85105**

Bemerkungen:
Die Akte enthält technische Angaben, die nach dem Eingang der Anmeldung eingereicht wurden und die nicht in dieser Patentschrift enthalten sind.

EP 1 658 042 B2

## Beschreibung

Die vorliegende Erfindung betrifft einerseits Bleichmittel für keratinhaltige Fasern mit einem pH-Wert von 4.5 bis 7,0, enthaltend Peroxoverbindungen und mindestens ein Alkalisierungsmittel ausgewählt aus Ammonium-, Alkalimetall- und Erdalkalimetall-carbonaten, -hydrogencarbonaten und -carbamiden, sowie Wasserstoffperoxid und $SiO_2$-Verbindungen, wobei letztgenannte gegebenenfalls hydratisiert sein können und andererseits die Verwendung von gegebenenfalls hydratisierten $SiO_2$-Verbindungen zur Steigerung der Aufhelleistung von Bleichmitteln für keratinhaltige Fasern sowie ein Verfahren zur Aufhellung keratinhaltiger Fasern mit den erfindungsgemäßen Bleichmitteln.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Aufhellen bzw. Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die oben genannte Anwendungsmischung wird im weiteren als "Bleichmittel" bezeichnet. Alle aufgeführten Mengenangaben beziehen sich, soweit nicht anders ausgeführt, ausschließlich auf diese Anwendungsmischungen.

Die herkömmlichen gebrauchsfertigen Bleichmittel für keratinhaltige Fasern enthalten neben Wasserstoffperoxid meist Peroxodisulfatverbindungen zur Steigerung der Aufhelleistung und besitzen bei der Anwendung auf der Faser einen pH-Wert von größer pH 9. Die Aufhelleistung ist bei diesem basischen pH-Wert optimal. Allerdings treten bei diesen Bedingungen einerseits Schädigungen der keratinhaltigen Faser und andererseits bei einer Anwendung am Probanden Hautirritationen auf. Eine Senkung des pH-Werts geht mit einer geringeren Haarschädigung und Hautirritation, aber auch zwangsläufig mit einer Verringerung der Aufhelleistung einher.

Die Offenlegungsschrift JP-A-04 279514 betrifft Haarbleichmittel, die bei einem pH-Wert von 5 bis 8 mild auf die keratinhaltige Faser und die Haut einwirken. Diese Bleichmittel enthalten Wasserstoffperoxid, Persulfat und Hydrogencarbonat. Allerdings genügt die Aufhelleistung dieser Bleichmittel nicht den Anforderungen an ein leistungsstarkes Bleichmittel.

Die Anmeldung WO-A1-01/47486 betrifft die Verwendung von ggf. hydratisierten $SiO_2$-Verbindungen zur Verringerung der Schädigung keratinischer Fasern durch oxidative Prozesse. Insbesondere werden oxidative Färbemittel, Haarbleichmittel sowie Fixiermittel im Rahmen einer Dauerwelle als Ausführungsform explizit genannt. Aus der WO-A1-01/47486 kann der Fachmann jedoch keinerlei Informationen zu speziellen Alkalisierungsmitteln für bestimmte pH-Bereiche entnehmen.

Aus EP 1219285 A2 sind oxidative Mittel zum Färben und Aufhellen von keratinischen Fasern bekannt, welche einen pH-Wert von 7,5 bis 12 besitzen und eine bestimmte Lösungsmittelkombination in Gegenwart von einer bestimmten Menge an Alkalisierungsmittel und Oxidationsmittel enthalten. Die in EP 1219285 A2 genannten Alkalisierungsmittel umfassen lediglich organische Alkalisierungsmittel, insbesondere Alkanolamine und Guanidinium-Salze.

Aufgabe der vorliegenden Erfindung ist es daher, die Aufhelleistung von Bleichmitteln für keratinhaltige Fasern, insbesondere in einem pH-Bereich von 4.5 bis 7,0, zu verbessern, um leistungsfähigere Bleichmittel mit erhöhter Verträglichkeit für die keratinhaltige Faser und die Haut des Probanden bereitzustellen.

Es wurde nun überraschenderweise gefunden, daß sich die Aufhelleistung von Bleichmitteln für keratinhaltige Fasern, insbesondere im pH-Bereich von 4.5 bis 7,0, durch den Zusatz von gegebenenfalls hydratisierten $SiO_2$-Verbindungen der Formel $(SiO_2)_n(Na_2O)_m(K_2O)_p$ steigern lässt, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

Ein erster Gegenstand der vorliegenden Erfindung ist daher die Verwendung von gegebenenfalls hydratisierten $SiO_2$-Verbindungen zur Steigerung der Aufhelleistung von Bleichmitteln für keratinhaltige Fasern, insbesondere in einem pH-Bereich von pH 4.5 bis 7.0, wobei die gegebenenfalls hydratisierte $SiO_2$-Verbindung ein Silikat der Formel

$(SiO_2)_n(Na_2O)_m(K_2O)_p$ ist, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

Unter keratinhaltigen Fasern werden erfindungsgemäß Pelze, Wolle, Federn und insbesondere menschliche Haare verstanden.

Erfindungsgemäß sind Wassergläser, die aus einem Silikat der Formel $(SiO_2)_n(Na_2O)_m(K_2O)_p$ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Ein zweiter Gegenstand der vorliegenden Erfindung sind Mittel zur Aufhellung keratinhaltiger Fasern mit einem pH-Wert von 4.5 bis 7,0, enthaltend mindestens eine Peroxoverbindung, Wasserstoffperoxid, mindestens ein Alkalisierungsmittel ausgewählt aus Ammonium-, Alkalimetall- und Erdalkalimetall-carbonaten, - hydrogencarbonaten und -carbamiden, sowie mindestens eine gegebenenfalls hydratisierte $SiO_2$-Verbindung der Formel $(SiO_2)_n(Na_2O)_m(K_2O)_p$, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

Die erfindungsgemäßen Bleichmittel enthalten als erste wichtige Komponente Wasserstoffperoxid. Das Wasserstoffperoxid wird erfindungsgemäß als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n $H_2O_2$ (n ist eine positive ganze Zahl größer 0), Harnstoffperoxid und Melaminperoxid, eingesetzt.

Obwohl bereits geringe Mengen der gegebenenfalls hydratisierten $SiO_2$-Verbindungen die Aufhelleistung erhöhen, kann es erfindungsgemäß bevorzugt sein, die gegebenenfalls hydratisierten $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.% bis 5 Gew.%, jeweils bezogen auf das gesamte erfindungsgemäße Mittel, einzusetzen. Die Mengenangaben geben dabei jeweils den Gehalt der $SiO_2$-Verbindungen (ohne deren Wasseranteil) in den Mitteln wieder.

Die Blondierwirkung des Wasserstoffperoxids kann zusätzlich durch sogenannte "Booster" gesteigert werden. Dies sind in der Regel Peroxoverbindungen, die keine Anlagerungsprodukte von Wasserstoffperoxid an andere Komponenten darstellen. Die Auswahl der in den erfindungsgemäßen Mitteln enthaltenen Peroxoverbindungen unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Magensium- und Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Peroxoverbindungen sind in den erfindungsgemäßen Bleichmitteln bevorzugt in Mengen von 1-40 Gew.%, insbesondere in Mengen von 2-30 Gew.-%, enthalten.

Die erfindungsgemäßen Bleichmittel werden bevorzugt kurz vor der Anwendung auf der Faser durch Vermengen von mindestens zwei Komponenten hergestellt. Zu diesem Zweck wird eine Komponente A, enthaltend mindestens eine Peroxoverbindung, mit einer Wasserstoffperoxid-Lösung als Komponente B vermischt, wobei die resultierende Mischung einen pH-Wert von 4.5 bis 7.0 besitzt. Die gegebenenfalls hydratisierten $SiO_2$-Verbindungen sind Bestandteil mindestens einer der Komponenten A und B. Sie sind jedoch bevorzugt in der Komponente A enthalten. Es kann bevorzugt sein, die Komponente A als Feststoff zu formulieren.

Die Komponente A wiederum kann jedoch auch aus einer Komponente A1 und einer Komponente A2 durch Mischung hergestellt werden. Dabei liegt die Komponente A1 bevorzugt in Form einer W/O- oder O/W-Emulsion und die Komponente A2 als Feststoff, enthaltend mindestens eine Peroxoverbindung, vor.

Die Konzentration der Wasserstoffperoxid-Lösung der Komponente B wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Komponente A und der Komponente B liegen dabei üblicherweise im Bereich 1:1 bis 1:2, wobei ein Überschuß an Komponente B insbesondere dann gewählt wird, wenn keine zu ausgeprägte Blondierwirkung erwünscht ist.

Das Alkalisierungsmittel ist bevorzugt in der Komponente A enthalten.

Die erfindungsgemäßen Bleichmittel enthalten die Alkalisierungsmittel bevorzugt in Mengen von 1 bis 25 Gew.%, insbesondere 1,5 bis 15 Gew.-%.

Als zusätzliche Bleichverstärker können die erfindungsgemäßen Mittel bevorzugt mindestens eine Verbindung, ausgewählt aus Essigsäure, Milchsäure, Weinsäure, Zitronensäure, Salicylsäure und ortho-Phthalsäure, enthalten.

In einer weiteren Ausführungsform enthalten die erfindungsgemäßen Bleichmittel zusätzlich strukturverbessernde Wirkstoffe. Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropan-diol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Mittel geeignet sind die Extrakte aus Mandel, Aloe Vera, Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1 - 10 Gew.-%, insbesondere 3 - 5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizen-proteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin® WQ (INCI-Bezeichnung: Laurdimonium Hydroxypropyl Hydrolyzed Wheat Protein) und Crotein® Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol® A15 (INCI-Bezeichnung: Polyquaternium-2), Onamer® M (INCI-Bezeichnung: Polyquaternium-1) und Merquat® 100 (INCI-Bezeichnung: Polyquaternium-6) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen, Hexosen und Heptosen, wie die entsprechenden On- und Uronsäuren (Zuckersäuren), Zuckeralkohole, Zuckeramine, wie beispielsweise N-Glucosamin, und Glykoside, sowie mit $C_4$-$C_{30}$-Fettalkoholen veretherte Pentosen, Hexosen und Heptosen, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Monobeziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure ist besonders bevorzugt. Weiterhin verbessern spezielle Phosphonsäuren und ihre Salze die Struktur keratinhaltiger Fasern. Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1 - 10 C-Atomen unter Öffnung des Epoxidrings und Ausbildung einer vizinalen Dihydroxyethyl- oder Hydroxy-alkoxy-ethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5 - 10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2 - 6 C-Atomen und 2 - 6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Bleichmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol®760 erhältlich.

Weiterhin kann als strukturverbessernder Wirkstoff Vitamin $B_3$ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3aS,4S, 6aR)-2-Oxohexa-hydrothienol[3,4-d]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di-und Oligosacchariden, Serin, Glycerinsäure, Niacinamid, Vitamin B6, Polyvinylpyrrolidon, Gluconsäure, Biotin und den lipidlöslichen Esteralkoholen oder Esterpolyolen.

Die erfindungsgemäßen Mittel enthalten die strukturverbessernden Wirkstoffe bevorzugt in Mengen von 0,1 bis 5 Gew.%, besonders bevorzugt in Mengen von 0,2 bis 2 Gew.-%.

In einer bevorzugten Ausführungsform der vorliegenden Erfindung enthalten die Mittel weiterhin eine Magnesiumverbindung. Die erfindungsgemäßen Mittel können durch Zugabe der $Mg^{2+}$-Kationen hinsichtlich ihre strukturerhaltenden Eigenschaften weiter optimiert werden. Bevorzugte Magnesiumverbindungen sind anorganische und organische $Mg^{2+}$-Salze, wie beispielsweise die Halogenide, die Carbonate und Hydrogencarbonate, das Acetat und das Citrat.

Die erfindungsgemäßen Mittel können weiterhin alle für solche Zubereitungen bekannten Wirk-, Zusatz- und Hilfsstoffe enthalten. In vielen Fällen enthalten die Bleichmittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x -CH_2COOH$, in der R eine lineare Alkylgruppe mit 10 bis 22 C-Atomen und $x = 0$ oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethytgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und $x = 0$ oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine $-COQ^{(-)}$-oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2--Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer $C_{8-18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N--Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolether-gruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

- $C_{12-22}$-Fettsäuremono- und -diester von Anlagerungsprodukten von 1 bis 30 Mol Ethylenoxid an Glycerin,
- $C_{8-22}$-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Bleichmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller. General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex®vertriebenen Methylhydroxyalkyldialkoyloxyalkylammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man- bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können - die erfindungsgemäßen Bleichmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat®280 (Dimethyldiallylammoniumchlorid-Acrylsäure-Copolymer im Handel erhältlichen Produkte sind Beispiele für solche kationischen Polymere.
- Copolymere des Vinylpyrrolidons mit quatemierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quatemierter Polyvinylalkohol - sowie die unter den Bezeichnungen

- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Potyquaternium-10 und Polyquaternium-22.

Als konditionierende Wirkstoffe weiterhin geeignet sind Silikonöle, insbesondere Dialkyl-und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxylamino-modifiziertes Silicon, das auch als Amodimethicone bezeichnet wird), SM-2059 (Hersteller. General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl, sowie Tocopherolacetat.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise

- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Cyclodextrine,
- Lösungsmittel und-vermittler wie Ethanol, Isopropanol, Ethylenglykol, Propylenglykol, Glycerin, Dimethylisosorbid und Diethylenglykol,
- quaternierte Amine wie Methyl-1-alkylamidoethyl-2-alkylimidazoliniummethosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, $\beta$-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,

- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein dritter Gegenstand der vorliegenden Erfindung ist ein Verfahren zur Aufhellung keratinhaltiger Fasern, bei dem die Fasern mit einem der oben beschriebenen Mittel behandelt werden.

Die folgenden Beispiele sollen den Gegenstand der vorliegenden Erfindung erläutern ohne ihn jedoch zu beschränken.

**Beispiele**

**1.0 Herstellung der Bleichmittel**

Es wurden als Komponente A folgende Cremegrundlagen A und B gemäß Tabelle 1 hergestellt:

| Tabelle 1 : | Cremegrundlage | Cremegrundlage |
|---|---|---|
| | **A** | **B** |
| (erfindungsgemäß) | | |
| Hydrenol® D[1] | 11.00 Gew.% | 11.00 Gew.% |
| Kokoslorol® C12-18[2] | 2.20 Gew.% | 2.20 Gew.% |
| Texapon® NSO F[3] | 26.50 Gew.% | 26.50 Gew.% |
| Ammoniumsulfat | 1.00 Gew.% | 1.00 Gew.% |
| Ammoniak (25 %ige wäßrige Lösung) | 7.60 Gew.% | 7.60 Gew.% |
| Gluadin® W40[4] | 0.35 Gew.% | 0.35 Gew.% |
| Natronwasserglas 40/42 | - | 2.00 Gew.% |
| Parfum | 0.90 Gew.% | 0.90 Gew.% |
| Wasser | ad 100 | ad 100 |

[1]$C_{16}$-$C_{18}$-Fettalkohol (INCI-Bezeichnung: Cetearyl alcohol) (COGNIS)

[2]$C_{12}$-$C_{18}$-Fettalkohol (INCI-Bezeichnung: Coconut alcohol) (COGNIS)

[3] Laurylethersulfat, Natriumsalz (ca. 27,5 % Aktivsubstanz; (INCI-Bezeichnung: Sodium Laureth Sulfate) (COGNIS)

[4] Weizenproteinhydrolysat (INCI-Bezeichnung: Aqua (Water), Hydrolyzed Wheat Protein, Sodium Benzoate, Phenoxyethanol, Methylparaben, Propylparaben) (COGNIS)

Vor der Anwendung am Haar wurden zu 50 g der Cremegrundlage A 12.5 g Ammoniumperoxodisulfat bzw. zu 50 g der Cremegrundlage B 12.5 g Ammoniumperoxodisulfat und 5.0 g Natronwasserglas 40/42 gemischt. Die beiden resultierenden Mischungen wurden jeweils mit 50 g der Komponente B gemäß Tabelle 2 unter Erhalt der Bleichmittel I bzw. II versetzt. Abschließend wurde der pH-Wert mit einer wäßrigen Lösung aus 50 Gew.% Zitronensäure auf pH 6.1 eingestellt.

| **Bleichmittel I** | **Bleichmittel II (erfindungsgemäß)** |
|---|---|
| 50 g Cremegrundlage A | 50 g Cremegrundlage B |
| 12.5 g Ammoniumperoxidisulfat | 12.5 g Ammoniumperoxidisulfat |
| | 5.0 g Natronwasserglas 40/42 |
| 50 g Komponente B | 50 g Komponente B |
| Zitronensäurelösung (ad pH 6.1) | Zitronensäurelösung (ad pH 6.1) |

(fortgesetzt)

| Bleichmittel I | Bleichmittel II (erfindungsgemäß) |
|---|---|
| Tabelle 2: | |
| Dipicolinsäure | 0.10 Gew.% |
| $Na_2H_2P_2O_7$ | 0.03 Gew.% |
| Turpinal® SL[5] | 1.50 Gew.% |
| Texapon®N 28[6] | 2.00 Gew.%. |
| Dow Corning DB 110[7] | 0.07 Gew.% |
| Aculyn® 33[8] | 12.00 Gew.% |
| Wasserstoffperoxid | 11.20 Gew.% |
| Ammoniak (25 %ige wäßrige Lösung) | 0.65 Gew.% |
| Wasser | ad 100 |

[5] 1-Hydroxyethan-1,1-diphosphonsäure (ca. 60% Aktivsubstanz; INCI-Bezeichnung: Etidronic Acid) (Cognis)

[6] Natriumlaurylethersulfat (ca. 28% Aktivsubstanz; INCI-Bezeichnung: Sodium Laureth Sulfate) (Cognis)

[7] nichtionogene Siliconemulsion (ca. 10% Aktivsubstanz; INCI-Bezeichnung: Dimethicone) (Dow Corning)

[8] wässrige Acrylatdispersion (ca. 28% Aktivsubstanz; INCI-Bezeichnung: Acrylates Copolymer) (Rohm & Haas)

## 2.0 Anwendung und farbmetrische Messungen

Der Grad der Aufhellung wurde an standardisierten Haarsträhnen (Alkinco 6634) durch farbmetrische Messungen der Fasern ermittelt. Die in Punkt 1.0 hergestellten Bleichmittel werden mit je einer Haarsträhne für 15 Minuten bei 32°C behandelt und danach mit Wasser gespült. Das Haar wurde abschließend getrocknet und mit dem Gerät Texflash DC 3881 der Firma Datacolor farbmetrisch zur Bestimmung der CIE-Lab-Werte vermessen.

Die Helligkeitsdifferenzen der aufgehellten Haarsträhne ergeben sich aus den jeweiligen CIE-Lab-Werten als $\Delta L$ und die gesamte Farbveränderung des Haars als Farbabstand $\Delta E$ nach der unten angeführten Farbabstandsformel (siehe auch DIN 6174, Deutsche Normen, Benth Verlag GmbH, Berlin, 1975). Als Referenz-Lab-Werte dienen die Werte des unbehandelten Ausgangshaars.

$$\Delta E = [(\Delta L)^2 + (\Delta a)^2 + (\Delta b)^2]^{1/2} \qquad \text{(Farbabstandsformel)}$$

In der Tabelle 3 sind die gemessenen CIE-Lab-Werte zusammengefasst. Je größer der $\Delta E$-Wert, umso größer ist die Farbänderung im Vergleich zum Ausgangshaar. Je größer der L-Wert bzw. der $\Delta L$-Wert, umso höher ist die Aufhelleistung.

| Tabelle 3 | | | | | |
|---|---|---|---|---|---|
| | L | a | b | $\Delta L$ | $\Delta E$ |
| **Bleichmittel A** | 45.63 | 45.63 | 24.01 | **10.68** | 15.44 |
| **Bleichmittel B (erfindungsgemäß)** | 54.92 | 11.48 | 28.56 | **19.97** | 25.26 |

Die Versuchsergebnisse beweisen, daß die Aufhelleistung eines Bleichmittels durch Zusatz von Natronwasserglas 40/42 als gegebenenfalls hydratisierte $SiO_2$-Verbindung um das 1.87-fache erhöht wird.

## Patentansprüche

1. Verwendung von gegebenenfalls hydratisierten $SiO_2$-Verbindungen zur Steigerung der Aufhellleistung von Bleichmitteln für keratinhaltige Fasern, insbesondere menschliche Haare, **dadurch gekennzeichnet, dass** die gegebenenfalls hydratisierte $SiO_2$-Verbindung ein Silikat der Formel $(SiO_2)_n(Na_2O)_m(K_2O)_p$ ist, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

2. Verwendung nach Anspruch 1, **dadurch gekennzeichnet, dass** die gegebenenfalls hydratisierte $SiO_2$-Verbindung eine wässrige Lösung des Silikats der Formel $(SiO_2)_n(Na_2O)_m(K_2O)_p$, in der n, m und p wie in Anspruch 1 definiert sind, ist.

3. Mittel zur Aufhellung keratinhaltiger Fasern, insbesondere menschlicher Haare, enthaltend mindestens eine Peroxoverbindung und Wasserstoffperoxid sowie mindestens ein Alkalisierungsmittel ausgewählt aus Ammonium-, Alkalimetall- und Erdalkalimetall-carbonaten, -hydrogencarbonaten und -carbamiden, **dadurch gekennzeichnet, dass** es

- zusätzlich mindestens eine gegebenenfalls hydratisierte $SiO_2$-Verbindung enthält, wobei die gegebenenfalls hydratisierte $SiO_2$-Verbindung ein Silikat der Formel $(SiO_2)_n(Na_2O)_m(K_2O)_p$ ist, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt,
- und einen pH-Wert von 4.5 bis 7.0 aufweist.

4. Mittel nach Anspruch 3, **dadurch gekennzeichnet, dass** die gegebenenfalls hydratisierte $SiO_2$-Verbindung eine wässrige Lösung des Silikats der Formel $(SiO_2)_n(Na_2O)_m(K_2O)_p$, in der n, m und p wie in Anspruch 3 definiert sind, ist.

5. Mittel nach einem der Ansprüche 3 bis 4, **dadurch gekennzeichnet, dass** es die gegebenenfalls hydratisierten $SiO_2$-Verbindungen in einer Menge von 0.05 bis 15 Gew.% enthält.

6. Mittel nach einem der Ansprüche 3 bis 5, **dadurch gekennzeichnet, dass** zusätzlich ein strukturverbessernder Wirkstoff, ausgewählt aus Panthenol, physiologisch verträglichen Panthenol-Derivaten, Mono-, Di- und Oligosacchariden, Serin, Glycerinsäure, Niacinamid, Vitamin B6, Polyvinylpyrrolidon, Gluconsäure, Biotin und den lipidlöslichen Esteralkoholen oder Esterpolyolen enthalten ist.

7. Mittel nach Anspruch 6, **dadurch gekennzeichnet, dass** es den strukturverbessernden Wirkstoff in Mengen von 0,1 bis 5 Gew.-% enthält.

8. Mittel nach einem der Ansprüche 3 bis 7, **dadurch gekennzeichnet, dass** die Peroxoverbindung ausgewählt wird aus Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Magnesiumperoxid und Bariumperoxid.

9. Mittel nach einem der Ansprüche 3 bis 8, **dadurch gekennzeichnet, dass** zusätzlich mindestens eine Säure, ausgewählt aus Essigsäure, Milchsäure, Weinsäure, Zitronensäure, Salicylsäure und ortho-Phthalsäure, enthalten ist.

10. Verfahren zur Aufhellung keratinhaltiger Fasern, **dadurch gekennzeichnet, dass** die Fasern mit einem Mittel nach einem der Ansprüche 3 bis 9 behandelt werden.

**Claims**

1. Use of optionally hydrated $SiO_2$ compounds for increasing the lightening power of bleaching agents for keratinic fibers, in particular human hair, **characterized in that** the optionally hydrated $SiO_2$ compound is a silicate of formula $(SiO_2)_n(Na_2O)_m(K_2O)_p$, wherein n represents a positive rational number and m and p represent independently of each other a positive rational number or 0, with the proviso that at least one of the parameters m or p is different from 0 and that the ratio between n and the sum of m and p lies between 1:4 and 4:1.

2. The use according to claim 1, **characterized in that** the optionally hydrated $SiO_2$ compound is an aqueous solution of the silicate of formula $(SiO_2)_n(Na_2O)_m(K_2O)_p$, wherein n, m and p are defined as in claim 1.

3. An agent for lightening keratinous fibers, in particular human hair, containing at least one peroxo compound, and hydrogen peroxide as well as at least one alkalinization agent selected from ammonium, alkaline metal and earth alkaline metal carbonates, hydrogen carbonates and carbamides, **characterized in that** it further has at least one optionally hydrated $SiO_2$ compound of formula $(SiO_2)_n(Na_2O)_m(K_2O)_p$, wherein n represents a positive rational number and m and p represent independently of each other a positive rational number or 0, with the proviso that at least one of the parameters m or p is different from 0 and that the ratio between n and the sum of m and p lies between 1:4 and 4:1, and a pH value from 4.5 to 7.0.

4. The agent according to claim 3, **characterized in that** the optionally hydrated $SiO_2$ compound is an aqueous solution of the silicate of formula $(SiO_2)_n(Na_2O)_m(K_2O)_p$, wherein n, m and p are as defined in claim 3.

5. The agent according to one of claims 3 to 4, **characterized in that** it contains the optionally hydrated $SiO_2$ compounds in an amount from 0.05 to 15% by weight.

6. The agent according to one of claims 3 to 5, **characterized in that**, additionally, a structure-enhancing active substance is contained, selected from panthenol, physiologically acceptable panthenol derivatives, mono-, di- and oligo-saccharides, serine, glyceric acid, niacinamide, vitamin B6, polyvinylpyrrolidone, gluconic acid, biotin and liposoluble ester alcohols or ester polyols.

7. The agent according to claim 6, **characterized in that** it contains the structure-enhancing active substance in amounts from 0.1 to 5% by weight.

8. The agent according to one of the claims 3 to 7, **characterized in that** the peroxo compound is selected from ammonium peroxodisulfate, potassium peroxodisulfate, sodium peroxodisulfate, ammonium persulfate, potassium persulfate, sodium persulfate, potassium peroxodiphosphate, magnesium peroxide and barium peroxide.

9. The agent according to one of the claims 3 to 8, **characterized in that**, additionally, at least one acid is contained, selected from acetic acid, lactic acid, tartaric acid, citric acid, salicylic acid and ortho-phthalic acid.

10. A method for lightening keratinous fibers, **characterized in that** the fibers are treated with an agent according to one of claims 3 to 9.

**Revendications**

1. Utilisation de composés de $SiO_2$ facultativement hydratés pour augmenter la puissance d'éclaircissement d'agents de blanchiment pour des fibres kératiniques en particulier des cheveux humains, **caractérisée en ce que** le composé de $SiO_2$ facultativement hydraté, est un silicate répondant à la formule $(SiO_2)_n(Na_2O)_m(K_2O)_p$ où n représente un nombre entier positif et m et p représentent, indépendamment l'un de l'autre, un nombre entier positif ou 0, avec ces mesures qu'au moins un des paramètres m ou p est différent de 0 et que le rapport entre n et la somme de m + p se situe entre 1:4 et 4:1.

2. Utilisation selon la revendication 1, **caractérisée en ce que** le composé de $SiO_2$ facultativement hydraté, est une solution aqueuse du silicate répondant à la formule $(SiO_2)_n(Na_2O)_m(K_2O)_p$ dans laquelle n, m et p ont les significations telles que définies à la revendication 1.

3. Agent pour l'éclaircissement de fibres kératiniques, en particulier de cheveux humains, contenant au moins un composé peroxo et du peroxyde d'hydrogène, ainsi qu'au moins un agent d'alcalinisation choisi parmi des carbonates, des hydrogénocarbonates et des carbamides d'ammonium, de métaux alcalins et de métaux alcalino-terreux, **caractérisé en ce qu'**il contient en outre au moins un composé de $SiO_2$ facultativement hydraté à la formule $(SiO_2)_n(Na_2O)_m(K_2O)_p$ où n représente un nombre entier positif et m et p représentent, indépendamment l'un de l'autre, un nombre entier positif ou 0, avec ces mesures qu'au moins un des paramètres m ou p est différent de 0 et que le rapport entre n et la somme de m + p se situe entre 1:4 et 4:1, et présente une valeur de pH de 4,5 à 7,0.

4. Agent selon la revendication 3, **caractérisé en ce que** le composé de $SiO_2$ facultativement hydraté, est une solution aqueuse du silicate répondant à la formule $(SiO_2)_n(Na_2O)_m(K_2O)_p$ dans laquelle n, m et p ont les significations telles que définies à la revendication 3.

5. Agent selon l'une quelconque des revendications 3 à 4, **caractérisé en ce qu'**il contient les composés de $SiO_2$

facultativement hydratés en une quantité de 0,05 à 15 % en poids.

6. Agent selon l'une quelconque des revendications 3 à 5, **caractérisé en ce qu'**il contient en outre une substance active structurante, choisie parmi le panthénol, des dérivés du panthénol physiologiquement acceptables, des monosaccharides, des disaccharides et des oligosaccharides, la sérine, l'acide glycérique, le niacinamide, la vitamine B6, la polyvinylpyrolidone, l'acide gluconique, la biotine et les esteralcools ou esterpolyols solubles dans les lipides.

7. Agent selon la revendication 6, **caractérisé en ce qu'**il contient la substance active structurante dans des quantités de 0,1 à 5 % en poids.

8. Agent selon l'une quelconque des revendications 3 à 7, **caractérisé en ce que** le composé peroxo est choisi parmi du peroxodisulfate d'ammonium, du peroxodisulfate de potassium, du peroxodisulfate de sodium, du persulfate d'ammonium, du persulfate de potassium, du persulfate de sodium, du peroxodiphosphate de potassium, du peroxyde de magnésium et du peroxyde de baryum.

9. Agent selon l'une quelconque des revendications 3 à 8, **caractérisé en ce qu'**il contient en outre au moins un acide choisi parmi l'acide acétique, l'acide lactique, l'acide tartrique, l'acide citrique, l'acide salicylique et l'acide orthophtalique.

10. Procédé pour l'éclaircissement de fibres kératiniques, **caractérisé en ce qu'**on traite les fibres avec un agent selon l'une quelconque des revendications 3 à 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- JP 4279514 A
- WO 0147486 A1
- EP 1219285 A2
- WO 9213829 A1
- DE 19522569
- DE 3725030 A
- DE 3723354 A
- DE 3926344 A

### In der Beschreibung aufgeführte Nicht-Patentliteratur

- **K. SCHRADER.** Grundlagen und Rezepturen der Kosmetika. Alfred Hüthig Verlag, 1989
- Kosmetik. Georg Thieme Verlag, 1995
- **KH. SCHRADER.** Grundlagen und Rezepturen der Kosmetika. Hüthig Buch Verlag, 1989
- DIN 6174. Benth Verlag GmbH, 1975